# EUROPEAN PATENT APPLICATION

(11) **EP 2 111 784 A2**
(43) Date of publication of application: **28.10.2009**
(21) Application number: 09156320.5
(22) Date of filing: 26.03.2009
(51) Int. Cl.: A61B 1/005

(54) **Bending portion of medical insertion apparatus, and manufacturing method and manufacturing apparatus thereof**

(30) Priority: 03.04.2008 JP 2008097431; 08.04.2008 US 43200 P
(71) Applicant: Olympus Corporation, Tokyo 151-0072 (JP)
(72) Inventor: Suigetsu, Naoki, Tokyo 192-8512 (JP); Kitagawa, Hideya, Tokyo 192-8512 (JP)
(74) Representative: Winter, Brandl, Fürniss, Hübner Röss, Kaiser, Polte Partnerschaft Patent- und Rechtsanwaltskanzlei

(57) **Abstract**

A manufacturing method for a bending portion of a medical insertion apparatus is characterized by comprising, preparing a bending tube (31) including cylindrical bending parts (32) coaxially connected to each other and rotatable with respect to each other, preparing a braid tube (34) including liner members made of a thermoplastic resin and forming a netlike shape, covering the braid tube (34) on an outer peripheral portion of the bending tube (31), pushing-heating of protruding inwardly a part of the braid tube (34) to be pushed into a push-in receiving portion (40) of the bending part (32a,32b) and heating the part to soften, and pushing-cooling of cooling the part to set.

## Description

The present invention relates to a bending portion of a medical insertion apparatus configured to be inserted into the interior of the body, and a manufacturing method and a manufacturing apparatus thereof.

Various medical insertion apparatuses configured to be inserted into the interior of the body has been used.

As such a medical insertion apparatus, there are an endoscope and an overtube for an endoscope for improving an insertion performance of the endoscope into the interior of the body.

In the endoscope and the overtube, an operation portion is connected to the proximal end portion of an elongate insertion portion, and the insertion portion is configured to be inserted into the interior of the body and the operation portion is configured to be held and operated by an operator. A bending portion is provided in the distal end portion of the insertion portion and configured to be actuated to be bent. The bending portion includes a bending tube forming a framework of the bending portion. The bending tube is formed of circularly cylindrical bending parts coaxially connected to each other and rotatable with respect to each other. The outer peripheral portion of the bending tube is covered with a braid tube. The braid tube is formed of strands made of metal or resin and braided into a circularly tubular shape. Both the end portions of the braid tube are fixed to both the end bending parts, respectively. The outer peripheral portion of the braid tube is covered with an outer tube.

Jpn. Pat. Appln. KOKAI Publication No. 11-206701 disclose that a braid tube made of metal is fixed to a bending part using soldering and a braid tube made of resin is fixed to a bending part by adhesive.

When a braid tube is fixed to a bending part using soldering or by adhesive as is disclosed in Jpn. Pat. Appln. KOKAI Publication No. 11-206701, a fixing work is very troublesome.

The present invention has been made in view of the above-mentioned problem, and is directed to providing a manufacturing method for a bending portion of a medical insertion apparatus wherein it is facilitated to fix a braid tube to a bending part, a bending portion of a medical insertion apparatus appropriate to be manufactured using the manufacturing method, and a manufacturing apparatus for a bending portion of a medical insertion apparatus appropriate to carry out the manufacturing method.

In an aspect of the present invention, a manufacturing method for a bending portion of a medical insertion apparatus is characterized by comprising; preparing a bending tube including cylindrical bending parts coaxially connected to each other and rotatable with respect to each other; preparing a braid tube including liner members made of a thermoplastic resin and forming a netlike shape; covering the braid tube on an outer peripheral portion of the bending tube; pushing-heating of protruding inwardly a part of the braid tube to be pushed into a push-in receiving portion of the bending part, and heating the part to soften; and pushing-cooling of cooling the part to set.

In the manufacturing method for the bending portion of the medical insertion apparatus according to the present aspect, the braid tube is fixed to the bending part just by protruding inwardly the part of the braid tube to be pushed into the push-in receiving portion of the bending part and heating the part to soften, and then cooling the part to set. Therefore, it is facilitated to fix the braid tube to the bending part.

In another aspect of the present invention, a bending portion of a medical insertion apparatus is characterized by comprising: a bending tube including cylindrical bending parts coaxially connected to each other and rotatable with respect to each other; and a braid tube covering an outer peripheral portion of the bending tube, and including liner members made of a thermoplastic resin and forming a netlike shape, and wherein the braid tube includes a push-in portion formed of a part of the braid tube and protruding inwardly and the bending tube includes a push-in receiving portion provided with the bending part and in which the push-in portion is pushed.

The bending portion of the medical insertion apparatus according to the present aspect is appropriate to be manufactured using the above manufacturing method for the bending portion of the medical insertion apparatus.

In a preferred aspect of the present invention, a manufacturing apparatus for a bending portion of a medical insertion apparatus, wherein the bending portion includes: a bending tube including cylindrical bending parts coaxially connected to each other and rotatable with respect to each other; and a braid tube covering an outer peripheral portion of the bending tube, and including liner members made of a thermoplastic resin and forming a netlike shape, is
characterized by comprising: a pushing-in instrument configured to protrude inwardly a part of the braid tube to be pushed into a push-in receiving portion of the bending part; and a pushing-in instrument heating mechanism configured to heat the pushing-in instrument.

The manufacturing apparatus for the bending portion of the medical insertion apparatus according to the present aspect is appropriate to carry out the above manufacturing method for the bending portion of the medical insertion apparatus.

In a preferred aspect of the present invention, the manufacturing apparatus for the bending portion of the medical insertion apparatus is characterized by further comprising a pushing-in instrument cooling mechanism configured to cool the pushing-in instrument.

The manufacturing apparatus for the bending portion of the medical insertion apparatus according to the present aspect is further appropriate to carry out the above manufacturing method for the bending portion of the medical insertion apparatus.

In a preferred aspect of the present invention, a manufacturing method for the bending portion of the medical insertion apparatus is characterized by further comprising: limiting-heating of limiting an outer diameter of the braid tube so as to be substantially equal to an outer diameter of the bending part in a region of the braid tube and heating the region to soften; and limitin-cooling of cooling the region to set.

In the manufacturing method for the bending portion of the medical insertion apparatus according to the present aspect, it is facilitated to form the limited portion whose outer diameter is limited so as to be substantially equal to the outer diameter of the bending part.

In a preferred aspect of the present invention, the bending portion of the medical insertion apparatus is characterized in that the braid tube includes a limited portion whose outer diameter of the limited portion is limited so as to be substantially equal to an outer diameter of the bending part.

The bending portion of the medical insertion apparatus according to the present aspect is appropriate to be manufactured using the above manufacturing method for the bending portion of the medical insertion apparatus.

In a preferred aspect of the present invention, the manufacturing apparatus for the bending portion of the medical insertion apparatus is characterized by further comprising: a limiting instrument configured to limit an outer diameter of the braid tube so as to be substantially equal to an outer diameter of the bending part in a region of the braid tube; and a limiting instrument heating mechanism configured to heat the limiting instrument.

The manufacturing apparatus for the bending portion of the medical insertion apparatus according to the present aspect is appropriate to carry out the above manufacturing method for the bending portion of the medical insertion apparatus.

In a preferred aspect of the present invention, the manufacturing apparatus for the bending portion of the medical insertion apparatus is characterized by further comprising a limiting instrument cooling mechanism configured to cool the limiting instrument.

The manufacturing apparatus for the bending portion of the medical insertion apparatus according to the present aspect is further appropriate to carry out the above manufacturing method for the bending portion of the medical insertion apparatus.

In a preferred aspect of the present invention, the manufacturing method for the bending portion of the medical insertion apparatus is characterized by further comprising: heating-fusing of heating the liner members to fuse in a region of the braid tube; cooling-joining of cooling the liner members fused by heating to set and to be joined to each other; cutting the braid tube in the region.

In the manufacture method for the bending portion of the medical insertion apparatus according to the present aspect, the liner members is joined to each other in the region of the braid tube and then the braid tube is cut in this region. Therefore, the liner members are prevented from being loosened in the cut portion.

In a preferred aspect of the present invention, the bending portion of the medical insertion apparatus is characterized in that the braid tube includes a joined portion provided on an end portion of the braid tube and in which the liner members are joined to each other.

The bending portion of the medical insertion apparatus according to the present aspect is appropriate to be manufactured using the above manufacturing method for the bending portion of the medical insertion apparatus.

In a preferred aspect of the present invention, the manufacturing apparatus for the bending portion of the medical insertion apparatus is characterized by further comprising: a joining instrument configured to join the liner members in a region of the braid tube; a joining instrument heating mechanism configured to heat the joining instrument; and a cutting mechanism configured to cut the braid tube in the region.

The manufacturing apparatus for the bending portion of the medical insertion apparatus according to the present aspect is appropriate to carry out the above manufacturing method for the bending portion of the medical insertion apparatus.

In a preferred aspect of the present invention, the manufacturing apparatus for the bending portion of the medical insertion apparatus is characterize by further comprising a joining instrument cooling mechanism configured to cool the joining instrument.

The manufacturing apparatus for the bending portion of the medical insertion apparatus according to the present aspect is further appropriate to carry out the above manufacturing method for the bending portion of the medical insertion apparatus.

In a preferred aspect of the present invention, the manufacturing method for the bending portion of the medical insertion apparatus is characterized by further comprising: limiting-heating and heating-fusing of limiting an outer diameter of the braid tube so as to be substantially equal to an outer diameter of the bending part in a region of the braid tube and heating the region to soften, and heating the liner members to fuse; limiting-cooling and cooling-joining of cooling the region to set, and cooling the liner members fused by heating to set and to be joined to each other; and cutting the braid tube in the region.

In the manufacturing method for the bending portion of the medical insertion apparatus according to the present aspect, the limiting-heating and the heating-fusing are carried out simultaneously with each other, and the limiting-cooling and the cooling-joining are carried out simultaneously with each other, thereby simplifying a manufacturing process and reducing manufacturing time.

In a preferred aspect of the present invention, the manufacturing apparatus for the bending portion of the medical insertion apparatus is characterized by further comprising: a limiting-joining instrument configured to limit an outer diameter of the braid tube so as to be substantially equal to an outer diameter of the bending part in a region of the braid tube and join the liner members in the region; a limiting-joining instrument heating mechanism configured to heat the limiting-joining instrument; and a cutting mechanism configured to cut the braid tube.

The manufacturing apparatus for the bending portion of the medical insertion apparatus according to the present aspect is appropriate to carry out the above manufacturing method for the bending portion of the medical insertion apparatus.

In a preferred aspect of the present invention, the manufacturing apparatus for the bending portion of the medical insertion apparatus is characterized by further comprising a limiting-joining instrument cooling mechanism configured to cool the limiting-joining instrument.

The manufacturing apparatus for the bending portion of the medical insertion apparatus according to the present aspect is further appropriate to carry out the above manufacturing method for the bending portion of the medical insertion apparatus.

In a preferred aspect of the present invention, the manufacturing method for the bending portion of the medical insertion apparatus is characterized in that the pushing-heating, and the limiting-heating and heating-fusing are carried out simultaneously with each other, and the pushing-cooling, and the limit-cooling and cooling-joining are carried out simultaneously with each other.

In the manufacturing method for the bending portion of the medical insertion apparatus according to the present aspect, the pushing-heating and the limiting-heating and heating-fusing are carried out simultaneously with each other, and the pushing-cooling and the limiting-cooling and cooling-joining are carried out simultaneously with each other, thereby simplifying a manufacturing process and reducing manufacturing time.

In a preferred aspect of the present invention, the manufacturing apparatus for the bending portion of the medical insertion apparatus is characterized by further comprising: a pushing-in and limiting-joining instrument including the pushing-in instrument and the limiting-joining instrument; and a pushing-in and limiting-joining instrument heating mechanism including a pushing-in instrument heating mechanism and a limiting-joining instrument heating mechanism.

The manufacturing apparatus for the bending portion of the medical insertion apparatus according to the present aspect is appropriate to carry out the above manufacturing method for the bending portion of the medical insertion apparatus.

In a preferred aspect of the present invention, the manufacturing apparatus for the bending portion of the medical insertion apparatus is characterized by further comprising a pushing-in and limiting-joining instrument cooling mechanism including the pushing-in instrument cooling mechanism configured to cool the pushing-in instrument and the limiting-joining instrument cooling mechanism configured to cool the limiting-joining instrument.

The manufacturing apparatus for the bending portion of the medical insertion apparatus according to the present aspect is further appropriate to carry out the above manufacturing method for the bending portion of the medical insertion apparatus.

In a preferred aspect of the present invention, the manufacturing method for the bending portion of the medical insertion apparatus is characterized by further comprising filling-setting of filling a filler into a part having a concave shape of the braid tube from outside to set.

In the manufacturing method for the bending portion of the medical insertion apparatus according to the present aspect, it is facilitated to form a shape keeping portion keeping the shape of the push-in portion formed of the part of the braid tube, protruding inwardly, and pushed in the push-in receiving portion.

In a preferred aspect of the present invention, the bending portion of the medical insertion apparatus is characterized in that the push-in portion has a concave shape, and the braid tube includes a shape keeping portion provided within the push-in portion and keeping the shape of the push-in portion.

The bending portion of the medical insertion apparatus according to the present aspect is appropriate to be manufactured using the above manufacturing method for the bending portion of the medical insertion apparatus.

In a preferred aspect of the present invention, the manufacturing apparatus for the bending portion of the medical insertion apparatus is characterized by further comprising a filling mechanism configured to fill a filler.

The manufacturing apparatus for the bending portion of the medical insertion apparatus according to the present aspect is appropriate to carry out the above manufacturing method for the bending portion of the medical insertion apparatus.

In a preferred aspect of the present invention, the manufacturing method for the bending portion of the medical insertion apparatus is characterized in that the filler is a thermoplastic adhesive.

In the manufacturing method for the bending portion of the medical insertion apparatus according to the present aspect, the thermoplastic adhesive is used as the filler, and therefore, it is possible to easily and smoothly carry out the filling-setting.

In a preferred aspect of the present invention, the manufacturing apparatus for the bending portion of the medical insertion apparatus is characterized in that the filling mechanism includes a thermoplastic adhesive filling mechanism configured to fill a thermoplastic adhesive.

The manufacturing apparatus for the bending portion of the medical insertion apparatus according to the present aspect is appropriate to carry out the above manufacturing method for the bending portion of the medical insertion apparatus.

In a preferred aspect of the present invention, a manufacturing method for an endoscope is characterized by comprising the above manufacturing method for the bending portion of the medical insertion apparatus.

The manufacturing method for the endoscope according to the present aspect includes the similar effects to those of the above manufacturing method for the bending portion of the medical insertion apparatus.

In a preferred aspect of the present invention, an endoscope is characterized by comprising the above bending portion of the medical insertion apparatus.

The endoscope according to the present aspect is appropriate to be manufactured using the above manufacturing method for the endoscope.

In a preferred aspect of the present invention, a manufacturing apparatus for an endoscope is characterized by comprising the manufacturing apparatus for the bending portion of the medical insertion apparatus.

The manufacturing apparatus for the endoscope according to the present aspect is appropriate to carry out the above manufacturing method for the endoscope.

In a preferred aspect of the present invention, a manufacturing method for an overtube for an endoscope is characterized by comprising the above manufacturing method for the bending portion of the medical insertion apparatus.

The manufacturing method for the overtube for the endoscope according to the present aspect includes the similar effects to those of the above manufacturing method for the bending portion of the medical insertion apparatus.

In a preferred aspect of the present invention, an overtube for an endoscope is characterized by comprising the above bending portion of the medical insertion apparatus.

The overtube for the endoscope according to the present aspect is appropriate to be manufactured using the above manufacturing method for the overtube for the endoscope.

In a preferred aspect of the present invention, a manufacturing apparatus for an overtube for an endoscope is characterized by comprising the above manufacturing apparatus for the bending portion of the medical insertion apparatus.

The manufacturing apparatus for the overtube for the endoscope according to the present aspect is appropriate to carry out the above manufacturing method for the overtube for the endoscope.

The invention can be more fully understood from the following detailed description when taken in conjunction with the accompanying drawings, in which:
FIG. 1 is a schematic view showing an endoscope according to a first embodiment of the present invention;
FIG. 2 is a perspective view showing a bending tube according to the first embodiment of the present invention;
FIG. 3 is a perspective view showing the bending tube and a braid tube according to the first embodiment of the present invention;
FIG. 4 is a longitudinal sectional view showing the bending tube and the braid tube according to the first embodiment of the present invention;
FIG. 5 is a perspective view showing a manufacturing apparatus according to the first embodiment of the present invention;
FIG. 6A is a perspective view showing a pushing-heating step, and a pushing-cooling step according to the first embodiment of the present invention;
FIG. 6B is a perspective view showing a pulling-out step according to the first embodiment of the present invention;
FIG. 7 is a perspective view showing a bending tube and a braid tube according to a second embodiment of the present invention;
FIG. 8A is a perspective view showing a preparing step according to the second embodiment of the present invention;
FIG. 8B is a perspective view showing a limiting-heating and heating-fusing step, and a limiting-cooling and cooling-joining step according to the second embodiment of the present invention;
FIG. 8C is a perspective view showing a separating step according to the second embodiment of the present invention;
FIG. 8D is a perspective view showing a cutting step according to the second embodiment of the present invention;
FIG. 9A is a perspective view showing a preparing step according to a third embodiment of the present invention;
FIG. 9B is a perspective view showing a pushing-heating, and limiting-heating and heating-fusing step, and a pushing-cooling, and a limiting-cooling and cooling-joining step according to the third embodiment of the present invention;
FIG. 9C is a perspective view showing a pulling-out and separating step according to the third embodiment of the present invention;
FIG. 10 is a longitudinal sectional view showing a bending tube and a braid tube according to a forth embodiment of the present invention;
FIG. 11A is a perspective view showing a early half of a filling step according to the forth embodiment of the present invention;
FIG. 11B is a perspective view showing an after half of the filling step according to the forth embodiment of the present invention;
FIG. 12 is a schematic view showing an overtube for an endoscope according to fifth embodiment of the present invention;
FIG. 13 is a perspective view showing a bending tube according to the fifth embodiment of the present invention;
FIG. 14 is a perspective view showing the bending tube and a braid tube according to the fifth embodiment of the present invention; and
FIG. 15 is a longitudinal sectional view showing the bending tube and the braid tube according to the fifth embodiment of the present invention.

Each embodiment of the present invention will hereinafter be described with reference to the drawings.

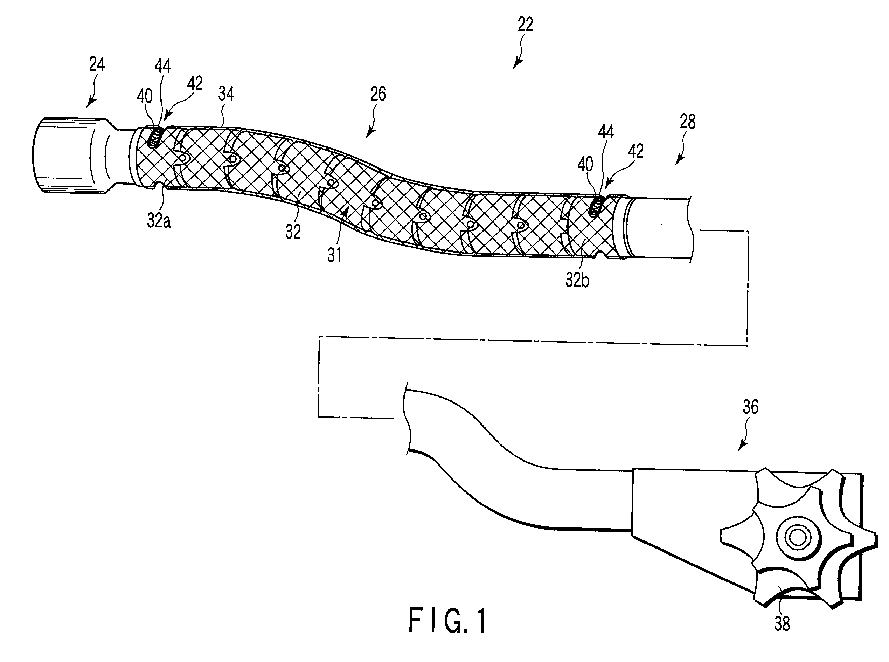

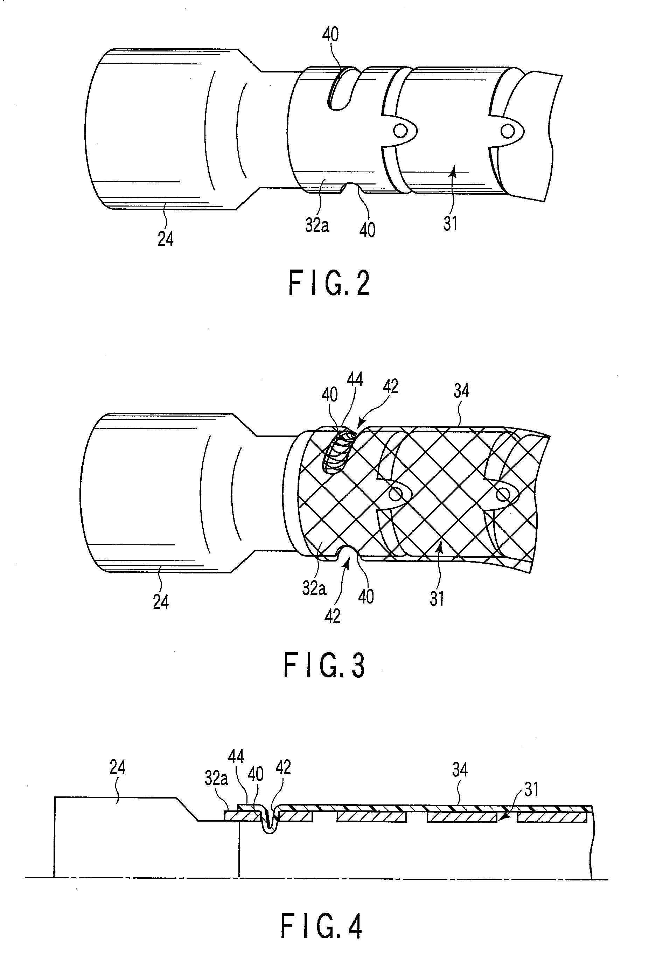

FIGS. 1 to 6B show first embodiment of the present invention.

Referring to FIG. 1, a schematic configuration of an endoscope as a medical insertion apparatus will be described.

The endoscope includes an elongate endoscope insertion portion 22 configured to be inserted into the interior of the body. In the endoscope insertion portion 22, a distal end rigid portion 24, an endoscope bending portion 26 to be actuated to be bent, a long flexible endoscope insertion tube portion 28 is provided from the distal end side to the proximal end side. The endoscope bending portion 26 includes a bending tube 31 forming a framework of the endoscope bending portion 26. The bending tube 31 is formed of substantially circularly cylindrical bending parts 32 coaxially connected to each other and rotatable with respect to each other. The outer peripheral portion of the bending tube 31 is covered with a braid tube 34. The braid tube 34 is formed of strands as liner members made of a thermoplastic resin and braided into a circularly cylindrical shape. Both end portions of the braid tube 34 are fixed to the bending parts 32a and 32b at both ends of the bending tube 31 and form the fixed end portions 44, respectively. The outer peripheral portion of the braid tube 34 is covered with an outer tube made of resin. An endoscope operation portion 36 is connected to the proximal end portion of the endoscope insertion portion 22 and configured to be held and operated. A bending operation knob 38 is provided in the endoscope operation portion 36 and configured to operate the endoscope bending portion 26 to be bent.

Referring to FIGS. 1 to 4, a fixing mechanism fixing the bending parts 32a and 32b and the braid tube 34 to each other will be described.

The bending parts 32a and 32b at both ends of the bending tube 31 includes circularly cylindrical body portion, respectively. A through groove 40 as a push-in receiving portion peripherally extends in the body portion of the bending part 32a, 32b. In the present embodiment, the two through grooves 40 are located symmetrically with respect to the central axis of the bending part 32a, 32b. On the other hand, the braid tube 34 includes a circularly tubular body portion. A protruding portion 42 as a push-in portion is formed of a part of the body portion of the braid tube 34 in each of the fixed end portions 44 at both ends of the braid tube 34, and radially inwardly protrudes and peripherally extends. In the present embodiment, the two protruding portions 42 is located symmetrically with the central axis of the braid tube 34. Then, each of the protruding portions 42 of the braid tube 34 is pushed in each of the through grooves 40 of the bending parts 32a and 32b. In this way, each of the fixed end portions 44 at both ends of the braid tube 34 is fixed to each of the bending parts 32a and 32b at both ends of the bending tube 31.

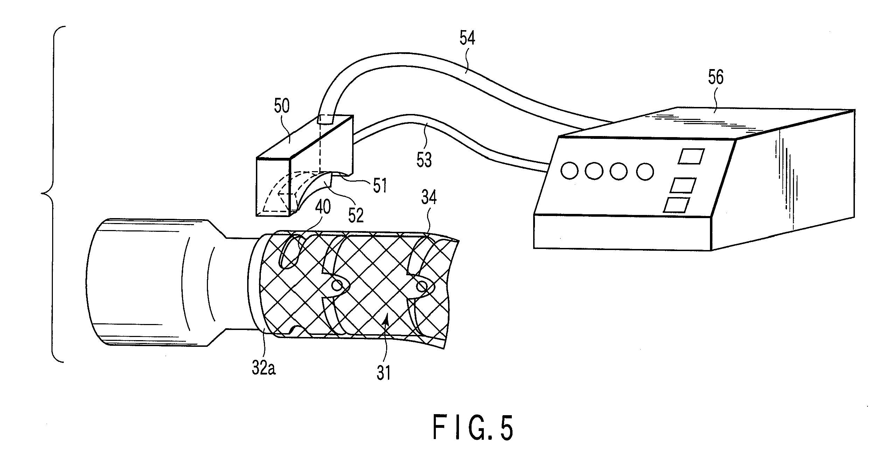

Referring to FIG. 5, a manufacturing apparatus for the endoscope bending portion will be described.

The manufacturing apparatus includes a pushing-in instrument 50. A placement portion 51 is formed in the pushing-in instrument 50 and has a shape of a circular arc whose inner diameter is large than the outer diameter of the bending part 32a, 32b. A forming portion 52 protrudes from the central portion of the placement portion 51, and has a shape corresponding with the shape of the protruding portion 42 of the braid tube 34 and slightly smaller than that. The pushing-in instrument 50 is configured to be located such that the forming portion 52 faces the through groove 40 of the bending part 32a, 32b and be movable in the radial direction of the bending part 32a, 32b.

A heater is built in the protruding forming portion 52 of the pushing-in instrument 50 and is connected to a power supply portion of an apparatus body 56 through an electric cable 53. The protruding forming portion 52 of the pushing-in instrument 50 is configured to be instantaneously heated up to a softening temperature at which the thermoplastic resin of the braid tube 34 is softened, by supplying electric power to the heater from the power supply portion through the electric cable 53. The pushing-in instrument 50 is hollow, and the inner space of the pushing-in instrument 50 is fluidly communicated with the blowing tube 54. The blowing tube 54 extends from the pushing-in instrument 50 and is connected to a blowing portion of the apparatus body 56. The protruding forming portion 52 of the pushing-in instrument 50 is configured instantaneously cooled using an air cooling by blowing air into the inside of the pushing-in instrument 50 from the blowing portion through the blowing tube 54.

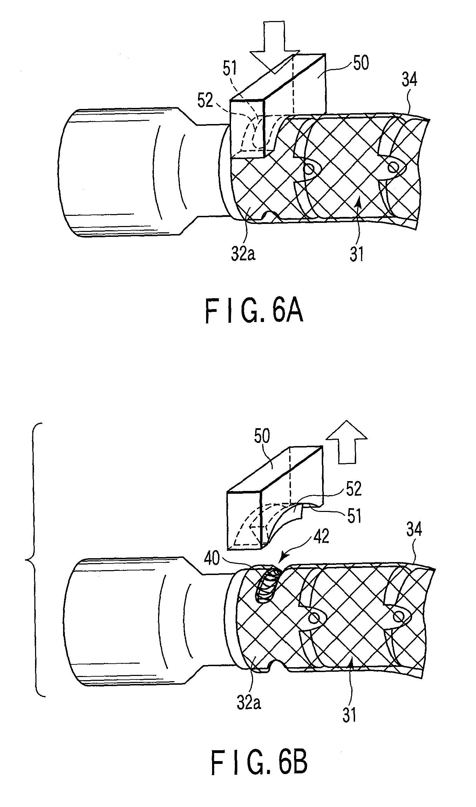

Referring to FIGS. 6A and 6B, a manufacturing method for the endoscope bending portion will be described.

As a thermoplastic resin for forming the braid tube 34 adapted for the manufacturing method according the present embodiment, polyamide and nylon, vinylon, polyester, polyethylene, and polypropylene, for example, is used, and polyethylene terephthalate out of polyester, and high density polyethylene out of polyethylene is preferably used.

### Preparing Step

Prepared is a bending tube 31 formed of substantially circularly cylindrical bending parts 32 coaxially connected to each other and rotatable with respect to each other and a braid tube 34 formed of strands made of a thermoplastic resin and braided into circularly tubular shape. A protruding portion 42 is not yet formed in the braid tube 34 at this preparing stage.

### Braid Tube Covering Step

The bending tube 31 is covered with the braid tube 34. Both ends of the braid tube 34 are positioned in both ends of the bending tube 31, respectively.

### Pushing-Heating Step

As is shown by an arrow in FIG. 6A, the pushing-in instrument 50 is radially inwardly moved so that the placement portion 51 of the pushing-in instrument 50 is placed on the braid tube 34, and the protruding forming portion 52 radially inwardly presses a part of the braid tube 34 so as to be push into a through groove 40 of the bending part 32a together with the protruding forming portion 52. The part of the braid tube 34 pushed in the through groove40 is referred to as a protruding portion preparing portion. After that, the protruding forming portion 52 of the pushing-in instrument 50 is instantaneously heated up to the softening temperature so that the protruding portion preparing portion of the braid tube 34 is instantaneously heated up to the softening temperature to soften. It is noted that the protruding forming portion 52 may be previously heated up to the softening temperature before the pushing-in step and may be heated simultaneously with the pushing-in step.

### Pushing-Cooling Step

Next, the protruding forming portion 52 of the pushing-in instrument 50 is instantaneously cooled so that the protruding portion preparing portion pushed in the through groove 40 and heated to soften is instantaneously cooled to set.

### Pulling-Out Step

As is shown by an arrow in FIG. 6B, the pushing-in instrument 50 is radially outwardly moved so that the protruding forming portion 52 of the pushing-in instrument 50 is pulled out from the through groove40 of the bending part 32a and the protruding portion 42 of the braid tube 34. Here, since the protruding forming portion 52 of the pushing-in instrument 50 is instantaneously heated and cooled so that the protruding portion preparing portion is instantaneously heated and cooled, the braid tube 34 is prevented from adhering to the protruding forming portion 52 and the outer surface thereof is finished to be smooth.

In this way, each of the protruding portions 42 pushed in each of the through grooves 40 of each of the distal end and the proximal end bending part 32a, 32b is formed in each of the distal end and the proximal end fixed end portion 44 of the braid tube 34, and each of the fixed end portions 44 is fixed to each of the bending parts 32a and 32b.

### Outer Tube Covering Step

The outer peripheral portion of the braid tube 34 is covered with an outer tube made of resin.

In the manufacturing method for the endoscope bending portion according to the present embodiment, the braid tube 34 is fixed to the bending part 32a, 32b just by radially inwardly protruding the part of the braid tube 34 to be pushed into the through groove 40 of the bending part 32a, 32b, heating the part to soften, and cooling the part to set. Therefore, it is facilitated to fix the braid tube 34 to the bending part 32a, 32b.

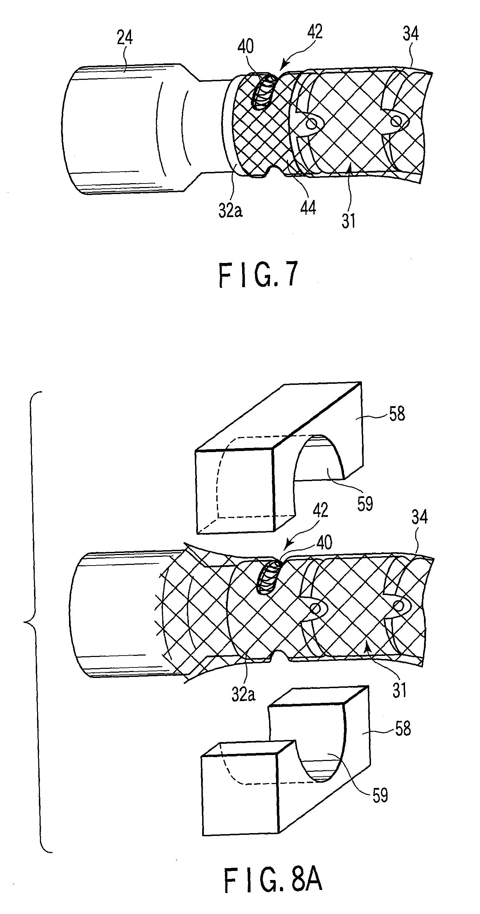

FIGS. 7 to 8D show a second embodiment of the present invention.

Referring to FIG. 7, as is similar to the first embodiment, each of protruding portions 42 of each of fixed end portions 44 at both ends of a braid tube 34 is pushed into each of through grooves 40 of each of bending parts 32a and 32b at both ends of a bending tube 31 so that each of the fixed end portions 44 is fixed to each of the bending parts 32a and 32b. Furthermore, in the fixed end portion 44 as a limited portion and a joined portion, the outer diameter of the braid tube 34 is limited so as to be substantially equal to the outer diameter of the bending part 32a, 32b and strands are joined to each other.

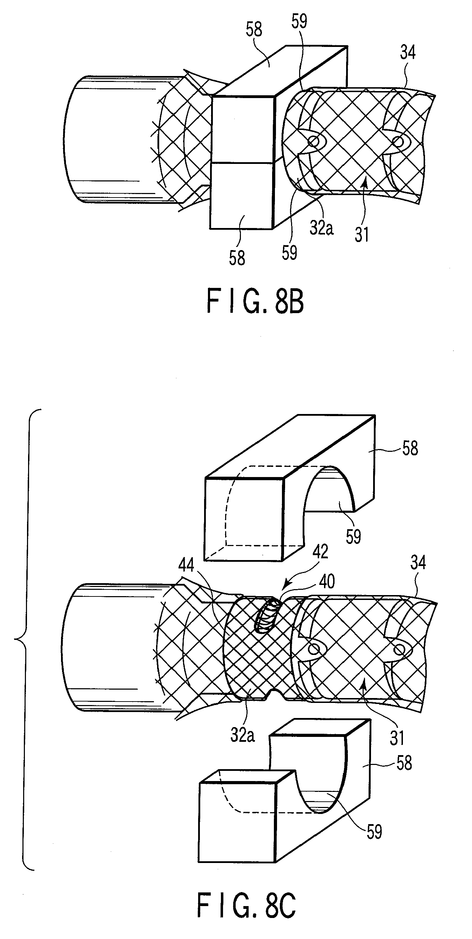

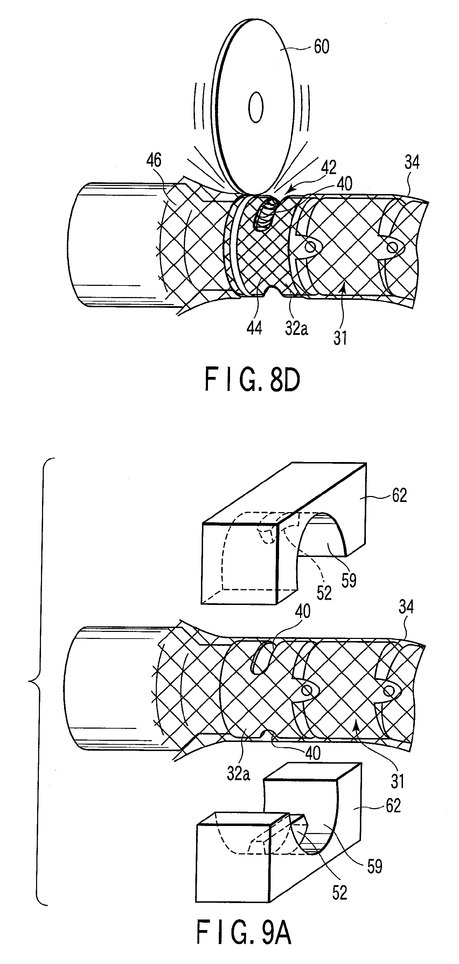

Referring to FIGS. 8A to 8D, a manufacturing apparatus for the endoscope bending portion will be described.

The manufacturing apparatus includes a pair of limiting-joining instruments 58 as well as a pushing-in instrument 50 similar to that of the first embodiment. A limiting-joining surface 59 is formed in the limiting-joining instrument 58 and has a shape of a semicircle whose inner diameter is equal to the outer diameter of the bending part 32a, 32b. The limiting-joining instruments 58 are configured to be located symmetrically with each other with respect to the central axis of the bending part 32a, 32b such that the central axes of the limiting-joining surfaces 59 are parallel with the central axis of the distal end or the proximal end bending part 32a, 32b and the limiting-joining surfaces 59 face the outer peripheral surface of the bending part 32a, 32b. Moreover, the limiting-joining instruments 58 are radially movable. When the limiting-joining instruments 58 are in contact with each other, the limiting-joining surfaces 59 form a circularly peripheral surface whose inner diameter is equal to the outer diameter of the bending part 32a, 32b. A heating mechanism and a cooling mechanism similar to those for the pushing-in instrument 50 are also used for the limiting-joining instrument 58, and the limiting-joining surface 59 of the limiting-joining instrument 58 is configured to be instantaneously heated up to a fusing temperature at which a thermoplastic resin is heated to fuse and is configured to be instantaneously cooled. Furthermore, the manufacturing apparatus includes a mechanical cutter 60 configured to cut the braid tube 34.

Referring to FIGS. 8A to 8D, a manufacturing method for the endoscope bending portion will be described.

### Preparing Step to Pulling-Out Step

As is similar to the first embodiment, a preparing step, a braid tube covering step, a pushing-heating step, a pushing-cooling step, a pulling-out step are carried out. However, in the braid tube covering step, both ends of a braid tube 34 is located so as to axially outwardly protrude beyond both ends of a bending tube 31, respectively.

### Limiting-Heating and Heating-Fusing Step

Referring to FIGS. 8A to 8B, regarding each of regions of the braid tube 34 covering each of a distal end and a proximal end bending part 32a, 32b, the limiting-joining instruments 58 is radially inwardly moved to hold the braid tube 34 and the bending part 32a, 32b between them, and the braid tube 34 is circumferentially pressed onto the peripheral surface of the bending part 32a, 32b so that the outer diameter of the braid tube 34 is limited so as to be substantially equal to the outer diameter of the bending part 32a, 32b. Hereinafter, the region of the braid tube 34 where the outer diameter is limited is referred to as the fixed end portion preparing portion. Next, the limiting-joining surfaces 59 are instantaneously heated up to the fusing temperature so that the fixed end portion preparing portion is instantaneously heated up to the fusing temperature. The fixed end portion preparing portion is heated to soften and a large number of strands in the fixed end portion preparing portion is heated to fuse. It is noted that the limiting-joining surface 59 may be previously heated up to the fusing temperature before the press onto the braid tube 34 and may be heated simultaneously with the press onto the braid tube 34.

### Limiting-Cooling and Cooling-Joining Step

Referring to FIG. 8B, the limiting-joining surfaces 59 of the limiting-joining instruments 58 are instantaneously cooled so that the fixed end portion preparing portion is instantaneously cooled to set and the large number of strands of the fixed end portion preparing portion is joined to each other.

### Separating Step

Referring to FIGS. 8B to 8C, the limiting-joining instruments 58 are radially outwardly moved so that the limiting-joining surfaces 59 are separated from the fixed end portion preparing portion. Here, since the limiting-joining surface 59 of the limiting-joining instrument 58 is instantaneously heated and cooled so that the fixed end portion preparing portion is instantaneously heated and cooled, the braid tube 34 is prevented from adhering to the limiting-joining surface 59 and the outer surface thereof is finished to be smooth. It is noted that unnecessary parts is left radially outwardly extended from the fixed end portion preparing portion in the braid tube 34.

### Cutting Step

The braid tube 34 is peripherally cut in the axial end portion of the fixed end portion preparing portion. Since the larger number of strands is joined to each other in the fixed end portion preparing portion, the strands are not loosened in the cut portion even if the cutting is carried out using the normal mechanical cutter 60.

In this way, the fixed end portion 44 is formed whose outer diameter is limited so as to be substantially equal to the outer diameter of the bending part 32a, 32b and in which the large number of strands is joined to each other.

In the manufacturing method for the endoscope bending portion according to the present embodiment, it is facilitated to form the fixed end portion 44 whose outer diameter is limited so as to be substantially equal to the outer diameter of the bending part 32a, 32b. Furthermore, since the axial end portion of the fixed end portion preparing portion is cut after the large number of strands is joined to each other in the fixed end portion preparing portion, the strands is prevented from being loosened in the cut portion. Moreover, the limiting-heating step and the heating-fusing step are carried out simultaneously with each other and the limiting-cooling step and the cooling-joining step are carried out simultaneously with each other, thereby simplifying a manufacturing process and reducing manufacturing time.

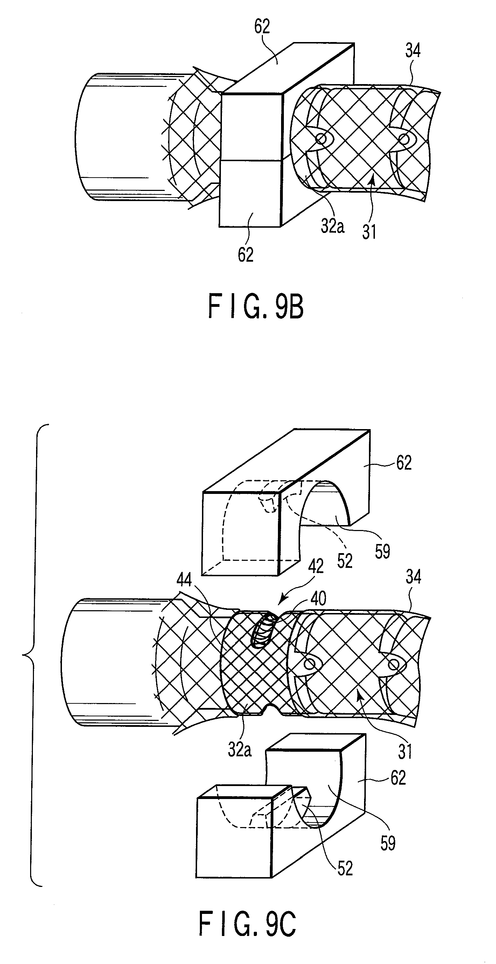

FIGS. 9A to 9C show a third embodiment of the present invention.

An endoscope bending portion 26 according to the present embodiment has the similar configuration as that of the endoscope bending portion 26 according to the second embodiment shown in FIG. 7.

Referring to FIG. 9A to 9C, a manufacturing apparatus for an endoscope bending portion will be described.

The manufacturing apparatus includes a pushing-limiting-joining instrument 62. In the pushing-limiting-joining instrument 62, the pushing-in instrument 50 (referring to FIG. 5) and the limiting-joining instrument 58 (referring to FIG. 8A) are integrated with each other. That is, in the pushing-limiting-joining instrument 62, the protruding forming portion 52 according to the first embodiment (referring to FIG. 5) protrudes from the limiting-joining surface 59 of the limiting-joining instrument 58 according to the second embodiment(referring to FIG. 8A).

Referring to FIGS. 9A to 9C, a manufacturing method for an endoscope bending portion will be described.

### Preparing Step

As is similar to the second embodiment, a bending tube and a braid tube is prepared.

Pushing-Heating, and Limiting-Heating and Heating-Fusing Step

Referring to FIGS. 9A to 9B, the pushing-limiting-joining instruments 62 are radially inwardly moved, so that the protruding forming portion 52 of the pushing-limiting-joining instrument 62 pushes a part of the braid tube 34 into a through groove 40 and the limiting-joining surfaces 59 limit the outer diameter of the braid tube 34 so as to be substantially equal to the outer diameter of the bending part 32a, 32b. Next, the protruding forming portion 52 of the pushing-limiting-joining instrument 62 is instantaneously heated up to the softening temperature and the limiting-joining surfaces 59 is instantaneously heated up to the fusing temperature so that a protruding portion preparing portion and a fixed end portion preparing portion of the braid tube 34 is heated to soften and a number of strands in the fixed end portion preparing portion is heated to fuse.

Pushing-Cooling, and Limiting-Cooling and Cooling-Joining Step

Referring to FIG. 9B, the protruding forming portion 52 and the limiting-joining surfaces 59 of the pushing-limiting-joining instrument 62 are instantaneously cooled so that the protruding portion preparing portion and the fixed end portion preparing portion are cooled to set and the number of strands in the fixed end portion preparing portion is joined to each other.

### Pulling-Out and Separating Step

Referring to FIGS. 9B to 9C, the pushing-limiting-joining instruments 62 are radially outwardly moved so that the protruding forming portions 52 of the pushing-limiting-joining instruments 62 are pulled out from the through grooves 40 of the bending part 32a, 32b and a protruding portion 42 of the braid tube 34, and the limiting-joining surfaces 59 are separated from the fixed end portion preparing portion.

### Cutting Step and Covering Step

As is similar to the second embodiment, a cutting step and a covering step is carried out.

In the manufacturing method for the endoscope bending portion according to the present embodiment, the pushing-heating step and the limiting-heating and heating-fusing step are carried out simultaneously with each other, the pushing-cooling step and the limiting-cooling and cooling-joining step are carried out simultaneously with each other, and the pulling-out step and the separating step are carried out simultaneously with each other, thereby simplifying a manufacturing process and reducing manufacturing time.

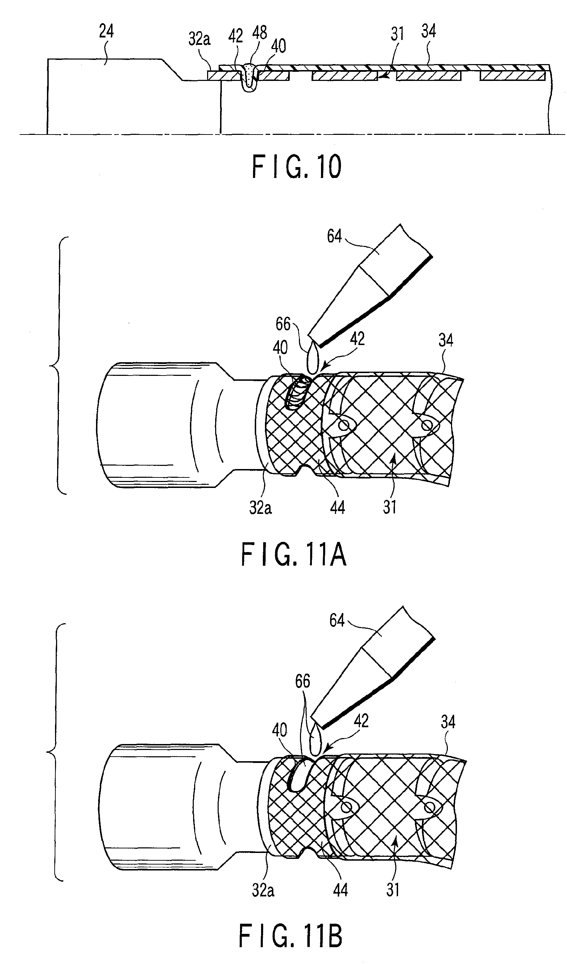

FIGS. 10 to 11B show a forth embodiment of the present invention.

Referring to FIG. 10, an endoscope bending portion 26 according to the present embodiment has the similar configuration to that of the endoscope bending portion 26 according to the second embodiment. In addition, a shape keeping portion 48 is formed within a substantially whole protruding portion 42 having a concave shape, of the braid tube 34 and keeps the shape of the protruding portion 42. The shape keeping portion 48 is formed of a thermoplastic adhesive 66 as a filler.

Referring to FIGS. 11A to 11B, a manufacturing apparatus for an endoscope bending portion will be described.

The manufacturing apparatus has the similar configuration to that of the manufacturing apparatus according to the second embodiment. In addition, the manufacturing apparatus includes a filling mechanism. The filling mechanism is configured to discharge the thermoplastic adhesive 66 from a filling nozzle 64.

Referring to FIGS. 11A to 11B, a manufacturing method for the endoscope bending portion will be described.

### Preparing Step to Cutting Step

As is similar to the second embodiment, a preparing step to a pulling-out step, a limiting-heating and heating-fusing step, a limiting-cooling and cooling-joining step, the separating step, and the cutting step is carried out.

### Filling Step

A thermoplastic adhesive 66 heated to fuse is discharged from the fill nozzle 64 and is filled into a protruding portion 42 having a concave shape of a braid tube 34. Next, the thermoplastic adhesive 66 is cooled to set. In this way, a shape keeping portion 48 keeping the shape of the protruding portion 42 is formed within the substantially whole protruding portion 42 having the concave shape, of the braid tube 34.

In the manufacturing method for the endoscope bending portion according to the present embodiment, the shape keeping portion 48 is formed just by filling the thermoplastic adhesive 66 into the protruding portion 42 having the concave shape and cooling the thermoplastic adhesive 66 to set. Therefore, it is facilitated to form the shape keeping portion 48.

Regarding each embodiment mentioned above, the endoscope bending portion according to each embodiment is appropriate to be manufactured using the manufacturing method according to each embodiment, respectively. The manufacturing apparatus according to each embodiment is also appropriate to carry out the manufacturing method according to each embodiment, respectively.

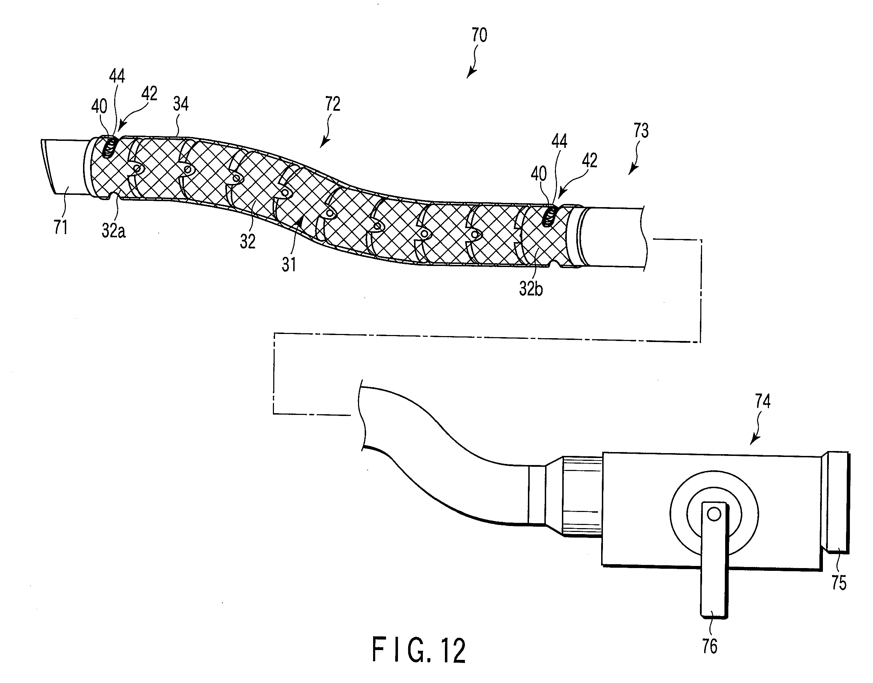

FIGS. 12 to 15 show a fifth embodiment of the present invention.

Referring to FIG. 12, a schematic configuration of an overtube for an endoscope as a medical insertion apparatus will be described.

The overtube improves an insertion performance of an endoscope into the interior of the body. In the overtube, a channel extends over the total length of the overtube, and an endoscope is configured to be inserted through the channel and movable forward and backward. In the distal end side portion of the overtube, an elongate tube insertion portion 70 is formed and is configured to be inserted into the interior of the body. In the tube insertion portion 70, a distal end projection and depression portion 71 forming an outlet of the channel of the overtube, the tube bending portion 72 configured to be actuated to be bent, a long flexible tube insertion tube portion 73 is provided form the distal end side to the proximal end side. Here, the distal end projection and depression portion 71 has a circularly cylindrical shape, and a distal end ring shape surface of the distal end projection and depression portion 71 is inclined with respect to the longitudinal axis of the tube insertion portion 70, and the endoscope is configured to project from and depress into a distal end inclined opening of the distal end projection and depression portion 71. The tube bending portion 72 has the similar configuration to that of the endoscope bending portion according to the first embodiment. That is, the tube bending portion 72 is formed of a bending tube 31 including bending parts 32, a braid tube 34 and an outer tube, and a distal end and a proximal end fixed end portion 44 of the braid tube 34 are fixed to bending parts 32a, 32b at the distal end and the proximal end of the bending tube 31. A tube operation portion 74 is connected to the proximal end portion of the tube insertion portion 70. A bending operation lever 76 for operating the tube bending portion 72 to be bent is provided in the tube operation portion 74. A proximal end insertion and pulling-out portion 75 is provided at the proximal end portion of the tube operation portion 74 and forms an inlet of the channel of the overtube. The endoscope is configured to be inserted into and pulled out from a proximal end opening of the proximal end insertion and pulling-out portion 75.

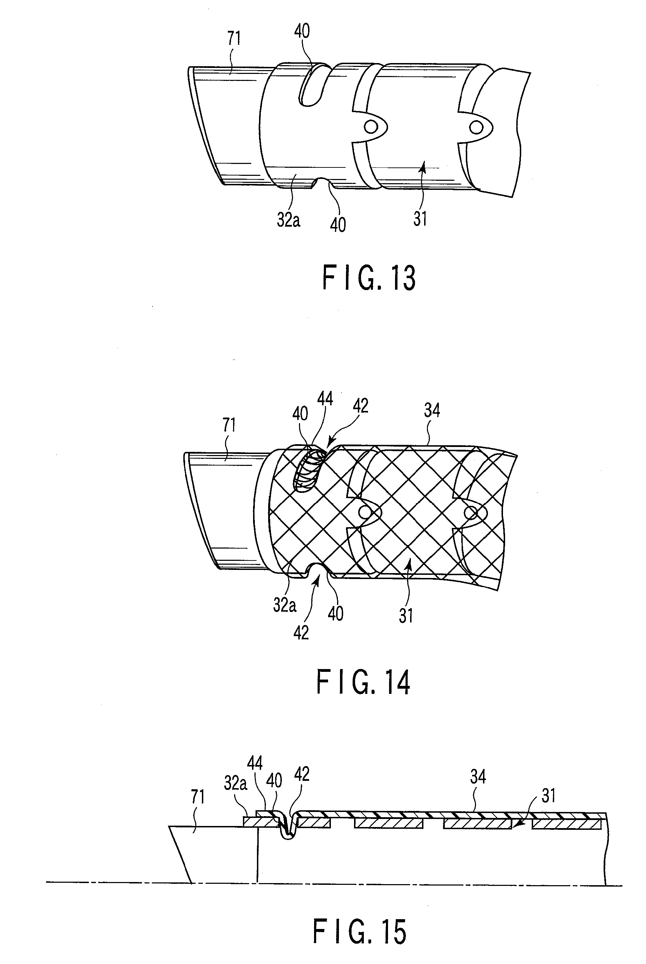

Referring to FIGS. 13 to 15, a fixing mechanism fixing the bending part 32a, 32b and the braid tube 34 will be described.

The fixing mechanism in the tube bending portion 72 is similar to the fixing mechanism of the endoscope bending portion according to the first embodiment. That is, in the tube bending portion 72, a protruding portion 42 as a push-in portion, of the fixed end portion 44 of the braid tube 34 is pushed in a through groove 40 as a push-in receiving portion, of the bending part 32a, 32b, and the braid tube 34 is fixed to the bending part 32a, 32b.

A manufacturing method and a manufacturing apparatus for a tube bending portion according to the present embodiment is similar to the manufacturing method and the manufacturing apparatus for the endoscope bending portion according to the first embodiment.

The tube bending portion 72, and the manufacturing method and a manufacturing apparatus thereof according to the present embodiment includes the similar effects to those of the endoscope bending portion, and the manufacturing method and the manufacturing apparatus thereof according to the first embodiment.

Moreover, a tube bending portion, and a manufacturing method and a manufacturing apparatus thereof similar to the endoscope bending portion, and the manufacturing method and the manufacturing apparatus according to any of the second to the forth embodiments may used.

## Claims

1. A bending portion of a medical insertion apparatus **characterized by** comprising:
a bending tube (31) including cylindrical bending parts (32a, 32b) coaxially connected to each other and rotatable with respect to each other; and
a braid tube (34) covering an outer peripheral portion of the bending tube (31), and including liner members made of a thermoplastic resin and forming a netlike shape, and
wherein the braid tube (34) includes a push-in portion (42) formed of a part of the braid tube (34) and protruding inwardly, and
the bending tube (31) includes a push-in receiving portion (40) provided with the bending part (32a, 32b) and in which the push-in portion (42) is pushed.

2. The bending portion of the medical insertion apparatus according to claim 1, **characterized in that** the braid tube (34) includes a limited portion (44) whose outer diameter of the limited portion (44) is limited so as to be substantially equal to an outer diameter of the bending part (32a, 32b).

3. The bending portion of the medical insertion apparatus according to claim 1, **characterized in that** the braid tube (34) includes a joined portion (44) provided on an end portion of the braid tube (34) and in which the liner members are joined to each other.

4. The bending portion of the medical insertion apparatus according to claim 1, **characterized in that** the push-in portion (42) has a concave shape, and the braid tube (34) includes a shape keeping portion (48) provided within the push-in portion (42) and keeping the shape of the push-in portion (42).

5. An endoscope **characterized by** comprising the bending portion of the medical insertion apparatus according to any one of claims 1 to 4.

6. An overtube for an endoscope **characterized by** comprising the bending portion of the medical insertion apparatus according to any one of claims 1 to 4.

7. A manufacturing method for a bending portion of a medical insertion apparatus, **characterized by** comprising;
preparing a bending tube (31) including cylindrical bending parts (32a, 32b) coaxially connected to each other and rotatable with respect to each other;
preparing a braid tube (34) including liner members made of a thermoplastic resin and forming a netlike shape;
covering the braid tube (34) on an outer peripheral portion of the bending tube (31);
pushing-heating of protruding inwardly a part of the braid tube (34) to be pushed into a push-in receiving portion(40) of the bending part (32a, 32b) and heating the part to soften; and
pushing-cooling of cooling the part to set.

8. A manufacturing method for the bending portion of the medical insertion apparatus according to claim 7, **characterized by** further comprising:
limiting-heating of limiting an outer diameter of the braid tube (34) so as to be substantially equal to an outer diameter of the bending part (32a, 32b) in a region of the braid tube (34) and heating the region to soften; and
limitin-cooling of cooling the region to set.

9. The manufacturing method for the bending portion of the medical insertion apparatus according to claim 7, **characterized by** further comprising:
heating-fusing of heating the liner members to fuse in a region of the braid tube (34);
cooling-joining of cooling the liner members fused by heating to set and to be joined to each other;
cutting the braid tube (34) in the region.

10. The manufacturing method for the bending portion of the medical insertion apparatus according to claim 7, **characterized by** further comprising:
limiting-heating and heating-fusing of limiting an outer diameter of the braid tube (34) so as to be substantially equal to an outer diameter of the bending part (32a, 32b) in a region of the braid tube (34) and heating the region to soften, and heating the liner members to fuse;
limiting-cooling and cooling-joining of cooling the region to set, and cooling the liner members fused by heating to set and to be joined to each other; and
cutting the braid tube (34) in the region.

11. The manufacturing method for the bending portion of the medical insertion apparatus according to claim 10, **characterized in that** the pushing-heating, and the limiting-heating and heating-fusing are carried out simultaneously with each other, and
the pushing-cooling, and the limit-cooling and cooling-joining are carried out simultaneously with each other.

12. The manufacturing method for the bending portion of the medical insertion apparatus according to claim 7, **characterized by** further comprising
filling-setting of filling a filler (66) into a part having a concave shape of the braid tube (34) from outside to set.

13. The manufacturing method for the bending portion of the medical insertion apparatus according to claim 12, **characterized in that**
the filler (66) is a thermoplastic adhesive(66).

14. A manufacturing method for an endoscope, **characterized by** comprising the manufacturing method for the bending portion of the medical insertion apparatus according to any one of claims 7 to 13.

15. A manufacturing method for an overtube for an endoscope, **characterized by** comprising the manufacturing method for the bending portion of the medical insertion apparatus according to any one of claims 7 to 13.

16. A manufacturing apparatus for a bending portion of a medical insertion apparatus,
wherein the bending portion includes: a bending tube (31) including cylindrical bending parts (32a, 32b) coaxially connected to each other and rotatable with respect to each other; and a braid tube (34) covering an outer peripheral portion of the bending tube (31), and including liner members made of a thermoplastic resin and forming a netlike shape, the manufacturing apparatus **characterized by** comprising:
a pushing-in instrument (50) configured to protrude inwardly a part of the braid tube (34) to be pushed into a push-in receiving portion (40) of the bending part (32a, 32b); and
a pushing-in instrument heating mechanism (53, 56) configured to heat the pushing-in instrument (50).

17. The manufacturing apparatus for the bending portion of the medical insertion apparatus according to claim 16, **characterized by** further comprising
a pushing-in instrument cooling mechanism (54, 56) configured to cool the pushing-in instrument (50).

18. The manufacturing apparatus for the bending portion of the medical insertion apparatus according to claim 16, **characterized by** further comprising:
a limiting instrument (58) configured to limit an outer diameter of the braid tube (34) so as to be substantially equal to an outer diameter of the bending part (32a, 32b) in a region of the braid tube (34); and
a limiting instrument heating mechanism (53, 56) configured to heat the limiting instrument (58).

19. The manufacturing apparatus for the bending portion of the medical insertion apparatus according to claim 18, **characterized by** further comprising
a limiting instrument cooling mechanism (54, 56) configured to cool the limiting instrument (58).

20. The manufacturing apparatus for the bending portion of the medical insertion apparatus according to claim 16, **characterized by** further comprising:
a joining instrument (58) configured to join the liner members in a region of the braid tube (34);
a joining instrument heating mechanism (53, 56) configured to heat the joining instrument (58); and
a cutting mechanism (60) configured to cut the braid tube (34) in the region.

21. The manufacturing apparatus for the bending portion of the medical insertion apparatus according to claim 20, characterize by further comprising
a joining instrument cooling mechanism (54, 56) configured to cool the joining instrument (58).

22. The manufacturing apparatus for the bending portion of the medical insertion apparatus according to claim 16, **characterized by** further comprising:
a limiting-joining instrument (58) configured to limit an outer diameter of the braid tube (34) so as to be substantially equal to an outer diameter of the bending part (32a, 32b) in a region of the braid tube (34) and join the liner members in the region;
a limiting-joining instrument heating mechanism (53, 56) configured to heat the limiting-joining instrument (58); and
a cutting mechanism (60) configured to cut the braid tube (34).

23. The manufacturing apparatus for the bending portion of the medical insertion apparatus according to claim 22, **characterized by** further comprising
a limiting-joining instrument cooling mechanism (54, 56) configured to cool the limiting-joining instrument (58).

24. The manufacturing apparatus for the bending portion of the medical insertion apparatus according to claim 22, **characterized by** further comprising:
a pushing-in and limiting-joining instrument (62) including the pushing-in instrument (50) and the limiting-joining instrument (58); and
a pushing-in and limiting-joining instrument heating mechanism (53, 56) including a pushing-in instrument heating mechanism (53, 56) and a limiting-joining instrument heating mechanism (53, 56).

25. The manufacturing apparatus for the bending portion of the medical insertion apparatus according to claim 24, **characterized by** further comprising
a pushing-in and limiting-joining instrument cooling mechanism (54, 56) including a pushing-in instrument cooling mechanism (54, 56) configured to cool the pushing-in instrument (50) and a limiting-joining instrument cooling mechanism (54, 56) configured to cool the limiting-joining instrument (58).

26. The manufacturing apparatus for the bending portion of the medical insertion apparatus according to claim 16, **characterized by** further comprising
a filling mechanism (64) configured to fill a filler (66).

27. The manufacturing apparatus for the bending portion of the medical insertion apparatus according to claim 26, **characterized in that**
the filling mechanism (64) includes a thermoplastic adhesive filling mechanism (64) configured to fill a thermoplastic adhesive (66).

28. A manufacturing apparatus for an endoscope, **characterized by** comprising the manufacturing apparatus for the bending portion of the medical insertion apparatus according to any one of claims 16 to 27.

29. A manufacturing apparatus for an overtube for an endoscope, **characterized by** comprising the manufacturing apparatus for the bending portion of the medical insertion apparatus according to any one of claims 16 to 27.
